# EUROPEAN PATENT SPECIFICATION

(11) **EP 4 371 548 B1**
(45) Date of publication and mention of the grant of the patent: **05.11.2025**
(21) Application number: 24166121.4
(22) Date of filing: 26.03.2019
(51) Int. Cl.: C04B 41/85

(54) **METHOD FOR APPLYING AN OPAQUE IMPARTING LIQUID FOR ZIRCONIA**
VERFAHREN ZUM AUFTRAGEN EINER OPAKEN VERMITTLUNGSFLÜSSIGKEIT FÜR ZIRKONIA
PROCÉDÉ D'APPLICATION D'UN LIQUIDE OPAQUE POUR LA ZIRCONE

(30) Priority: 30.03.2018 JP 2018066937; 18.03.2019 JP 2019049270
(43) Date of publication of application: 22.05.2024
(62) Divisional of application: 19165193.4
(73) Proprietor: Shofu Inc., Kyoto 605-0983 (JP)
(72) Inventor: KITAMURA, Toshio, KYOTO, 605-0983 (JP); TAKAHASHI, Shuhei, KYOTO, 605-0983 (JP)
(74) Representative: Eisenführ Speiser

(56) References cited:
- US-A1- 2013 115 365
- DATABASE WPI Week 201633, Derwent World Patents Index; AN 2015-81259V, XP002795190
- DATABASE WPI Week 201813, Derwent World Patents Index; AN 2018-06162T, XP002795191

## Description

### BACKGROUND OF THE INVENTION

### Field of the Invention

The present invention relates to an opaque imparting method comprising an opaque imparting liquid to exhibit opaque property on a zirconia ceramic prosthesis device after sintering by applying to the prosthesis device cut and machined from a dental zirconia for cutting and machining by using a CAD/CAM system in a dental field.

### Description of the Related Art

In the conventional dental treatment of a defect part of a dental crown, a prosthetic restoration using a casting crown bridge and an artificial tooth has been performed generally. Specific examples include a clinical application of a porcelain baked crown bridge which reproduces a tooth crown shape by baking porcelain material on a surface of a metal frame made from a casting alloy for porcelain baking and has both a functionality and aesthetic property.

In addition, from the point of view of the metal allergy and the price remarkable rise to depend on the noble metal market price and from the point of view of aesthetic property which can imitate a color tone of a natural tooth, a prosthesis device, which is so-called all ceramics, prepared by the dipping method using alumina, aluminosilicate glass, lithium disilicate glass and the like or by the press method using ceramic ingot, has attracted attention, and, the prosthesis restoration using it has been increased.

In recent years, techniques to prepare a prosthesis device by the cutting and machining which uses a dental CAD/CAM system spread rapidly and it has been becoming possible to easily prepare prosthetic devices by milling blanks such as a block and a disk which are made of zirconia, alumina, aluminosilicate glass, and lithium disilicate glass. In addition, although the zirconia has been frequently used in the clinical as high-strength ceramic, a zirconia blank (pre-sintered body) which has adjusted strength and hardness which are advantageous for cutting and machining and is prepared by pre-sintering (temporary calcined) at low sintering temperature is generally used. The zirconia blank which is the pre-sintered body is not subjected to the perfect sintering in order to improve cutting property in the zirconia blank.

Since this zirconia blank has the bending strength which is used in the frame of 4 or more units bridge, a tetragonal partially stabilized zirconia blank containing 3 mol% of yttrium oxide has been put to practical use. This zirconia blank containing 3 mol% of yttrium is added with a very small amount of alumina for improving sinterability and restraining the low temperature deterioration. In addition, in order to increase optical transparency and to use as a molar tooth full crown, a zirconia blank containing an infinitesimal alumina has been clinically used. In addition, in order to use as a front tooth full crown, a zirconia blank in which optical transparency is highly designed by increasing a content of yttrium oxide to 5-6 mol%, which is added as a stabilizer, also has been used.

Zirconia is broadly divided into two kinds including a white color tone zirconia and a colored zirconia where the color tone is toned to a color tone close to that of a natural tooth. In the color tone of the natural tooth, the color becomes darker from an incisal part corresponding to an enamel of the tooth toward a cervical portion, which is called a condition where the chroma increases.

Therefore, a technique to mold a dental zirconia blank for cutting and machining which has multilayered structure and is imparted with the gradation of the color prepared by superposing progressively a plurality of zirconia powders which have different color tones including a color tone corresponding to an enamel color of an incisal part and a color tone corresponding to a cervical portion color in a layer form in order to reproduce the color tones of the incisal part and the cervical portion of the natural tooth in a prosthesis device which is cut and machined from the dental zirconia blank has been used.

This dental zirconia blank for cutting and machining which has the multilayered structure has a feature that a plurality of layers having different color tones, optical transparency or the like are superposed at various thickness.

However, when an yttrium content of the zirconia blank is 5 mol% or more, although the transparency is improved, the strength decreases, and therefore there is a problem that breakage is caused when it is used for the case of 4 or more bridges.

On the other hand, when an yttrium content of the zirconia blank is within a range of 3 to 4 mol%, since sufficient transparency is not obtained, it is difficult to reproduce transparency corresponding to an enamel portion of a tooth. Therefore it is necessary to build a glass which is called as the porcelain to the enamel. However, this process is a very complicated process and requires the technique of the technician.

On the other hand, a trial to improve sintering property by incorporating 0.2 to 2 wt.% of alumina into a zirconia containing 3 mol % of yttrium for the purpose of improving strength has been performed. However, there is a problem that sufficient transparency is not obtained in the zirconia containing alumina.

As described above, the improvement of the translucency of a dental zirconia material to be close to the translucency of the natural tooth has been tried so far. However, the part called an abutment tooth used in a dentistry include a metal abutment, a discolored tooth or the like. In the case of a transparent zirconia, there is a problem that it is easily affected by the color tone of the abutment tooth. In the case of using an opaque zirconia, although the influence of an abutment tooth decreased, there is a problem that transparency of an enamel layer is not enough. Therefore, it has been necessary to having an opaque function which can impart slightly opacity only to the part of the abutment tooth.

On the other hand, there is a method for adding a color to a semi-sintered zirconia in imitation of an abutment tooth by a coloring material. Some of this method exhibit an opaque effect by applying water-soluble solution of titanium, however there is a problem that titanium causes cloudiness in sintering and properties are decreased.

Japanese Unexamined Patent Application Publication (Translation of PCT Application) No.2016-500955 discloses a preparing method of a zirconia having a color tone which is closes to the color tone of the tooth by applying a coloring material to a pre-sintered body of the zirconia. In this method, the transparency is improved by coloring a surface of the zirconia, however, the coloring material is used, it is impossible to adjust darkness for adjusting opacity.

US 2013/115365 A1 describes a method for changing a translucent property of zirconia dental materials.

### SUMMARY OF THE INVENTION

### Technical Problem

In the above described prior arts, although a coloring effect is exhibited, it is impossible to impart opacity only to a portion where an adjustment of transparency is desired. A problem to be solved by the present invention is to provide a liquid material which can only adjust transparency by applying on a part of a zirconia crown having high transparency, without coloring. In addition, a zirconia crown without decreasing strength after sintering is provided by applying this liquid material.

### Solution to Problem

The present invention provides an opaque imparting method as defined in appended claim 1. Preferred embodiments of the invention are set forth in claims 2 to 5.

The method of the invention comprises applying an opaque imparting liquid only to an inner surface of a prosthesis device cut and machined from a dental zirconia for cutting and machining, wherein the opaque imparting liquid contains
(a) 10 to 39 wt.% of a water-soluble aluminum compound and/or a water-soluble lanthanum compound,
(b) 60 to 89 wt.% of water, and
(c) 1 to 20 wt.% of an organic solvent.

It is preferable that the organic solvent (c) contains any of alcohols, polyol and glycol ethers. It is preferable that the organic solvent (c) is any of alcohols, polyol and glycol ethers.

It is preferable that the water-soluble aluminum compound and/or the water-soluble lanthanum compound (a) contains aluminum acetate and/or lanthanum acetate. It is preferable that the water-soluble aluminum compound and/or the water-soluble lanthanum compound (a) is aluminum acetate or lanthanum acetate.

It is preferable that the dental zirconia for cutting and machining is colored by a zirconia powder where iron, erbium, cobalt or the like is solid-solved.

In the opaque imparting method of the present invention, it is preferable that the prosthesis device cut and machined from the dental zirconia for cutting and machining is a crown or a bridge.

### Advantageous Effects of Invention

By applying the opaque imparting liquid to a prosthesis device cut and machined from a dental zirconia for cutting and machining, opaque property is partially imparted to the prosthesis device partially without coloring.

Furthermore, by applying to a pre-sintered body of a pre-colored zirconia, opaque property is imparted to only the applied portion and a masking effect for a discolored tooth is exhibited by adjusting application amount.

### DETAILED DESCRIPTION OF THE PREFERRED EMBODIMENTS

Hereinafter, constituent elements in the present invention are described in detail.

As the component (a) the water-soluble aluminum compound and/or the water-soluble lanthanum compound contained in the opaque imparting liquid, any aluminum compound may be used regardless of the degree of the solubility as long as it dissolves in water. Among them, specific examples include lactate, aluminum ethylenediamine tetraacetate, aluminum oxide tartrate hydrate, sodium aluminum citrate, potassium aluminum citrate, aluminum chloride oxide, aluminum citrate, oxy aluminum acetate, oxy aluminum perchlorate, oxy aluminum salicylate, aluminium trichloride, aluminium tribromide, aluminium hydroxide, oxy dialuminum carbonate, oxy aluminium nitrate, aluminium sulfate and aluminium nitrate, and ethylenediamine tetraacetate is particularly preferable. These aluminum compounds may be used alone or in combination of two or more thereof.

In addition, any lanthanum compound may be used regardless of the degree of the solubility as long as it dissolves in water. Preferable lanthanum compounds include lanthanum chloride, lanthanum nitrate, lanthanum sulfate and lanthanum acetate and lantern chloride and lanthanum acetate are particularly preferable.

It is essential that a content of the water-soluble aluminum compound and/or the water-soluble lanthanum compound contained in the opaque imparting liquid is within a range of 10 wt.% to 39 wt.% and the content is preferably within a range of 20 wt.% to 30wt.%. When the content of the water-soluble aluminum compound and/or the water-soluble lanthanum compound contained in the opaque imparting liquid is less than 10 wt.%, opaque property is not imparted even if the opaque imparting liquid is applied to a prosthesis device. On the other hand, when the content is more than 39 wt.%, because solubility to water decreases remarkably, it is impossible to prepare an uniform opaque imparting liquid.

The water (b) contained in the opaque imparting liquid is necessary for dissolution or dispersion of the water-soluble aluminum compound and/or the water-soluble lanthanum compound, and is advantageous for dissolution particularly.

The water contained in the opaque imparting liquid is not particular limited, but it is possible to use ion exchanged water, Japanese pharmacopeia purified water and Japanese pharmacopeia distilled water and the like. A content of the water contained in the opaque imparting liquid is within a range of 60 to 89 wt.% and is preferably within a range of 65 to 79 wt.%. When the content is little, the solubility of the water-soluble aluminum compound and/or the water-soluble lanthanum compound (a) decreases and when the content is large, the effect by imparting opaque property decreases.

As the organic solvent (c) contained in the opaque imparting liquid, an organic solvent compatible with the water (b) is appropriately selected and used. It is preferable that the organic solvent acts as a thickener to adjust a viscosity and does not generate a residue as an organic matter in sintering the zirconia. Specific examples of the organic solvent include methanol, ethanol, isopropanol, ethylene glycol, polyethylene glycol, acetone, 1,4-dioxane, polypropylene glycol. Among them, polyethylene glycol and polypropylene glycol are preferable. In addition, a content of the organic solvent contained in the opaque imparting liquid is within a range of 1 to 20 wt.%, and is preferably within a range of 1 to 5 wt.%. When the content of the organic solvent is less than 0.1 wt.%, application property decreases. When the content is more than 20 wt.%, liquid permeability decreases.

In addition, the opaque imparting liquid may contain an organic marker based on organic dye. When this opaque imparting liquid is applied to a prosthesis device prepared by cutting and machining a dental zirconia for cutting and machining, it is possible to visualize the applied area and to visualize the applied amount of the opaque imparting liquid and the degree of permeation thereof. It is required for the organic dye not to contain an inorganic matter. Specific examples of the organic dye include gardenia yellow pigment, annatto pigment and red cabbage pigment. Among them, red cabbage pigment and gardenia yellow pigment are preferable.

When the opaque imparting liquid is applied to a prosthesis device (semi-sintered body) prepared by cutting and machining a dental zirconia for cutting and machining which is porous and pre-sintered and the applied prosthesis device is perfect sintered, a chroma of a color tone and transparency are improved by the opaque imparting liquid. Therefore, the opaque imparting liquid may permeate a prosthesis device which is a pre-sintered body and impart opaque property to the prosthesis device by perfect sintering. In order to exhibit such an effect surely, it is preferable that relative density, specific surface area and composition of a dental zirconia for cutting and machining which is cut and machined for preparing a prosthesis device are adjusted.

Accordingly, it is preferable that the relative density of the a dental zirconia for cutting and machining which is cut and machined for preparing a prosthesis device is within a range of 45 to 60% (when the density of the final sintered body is 100%, the relative density means an apparent density of a semi-sintered body.). When the relative density is less than 45%, the machinability with the CAD/CAM machining machine may decrease. On the other hand, when the relative density is more than 60%, there may be a problem that the applied opaque imparting liquid is hard to permeate. In addition, it is preferable that the specific surface area of a dental zirconia blank for cutting and machining is within a range of 10 to 200 cm²/g from a point of view that a pre-sintered body is porous. When the specific surface area is less than 10 cm²/g, there is a case where the permeation of the opaque imparting liquid is not sufficient, and when the specific surface area is more than 200 cm²/g, there is a case where cutting property with the CAD/CAM machining machine decreases.

In addition, it is preferable that a dental zirconia for cutting and machining which is cut and machined for preparing a prosthesis device which is applied with the opaque imparting liquid contains yttrium and/or erbium as a stabilizing material. When the dental zirconia for cutting and machining which is cut and machined for preparing a prosthesis device which is applied with the opaque imparting liquid contains the stabilizing material, aesthetic property is increase. It is preferable that the molar concentration of the stabilizing material contained in the dental zirconia for cutting and machining is 4 mol% or more. It is preferable that the present invention is used for a zirconia having high transparency.

It is preferable that a dental zirconia for cutting and machining which is cut and machined for preparing a prosthesis device which is applied with the opaque imparting liquid is prepared by using colored zirconia powder. Specific examples include zirconia powder containing the above erbium stabilizing material (red) and yellow zirconia powder contained iron. In addition, there is no problem at all even if other colored zirconia powder which contains element such as cobalt (gray), manganese (red), chromic (yellow) for adjusting a color in addition to these colored zirconia powder is used together.

The kind of a prosthesis device applied with the opaque imparting liquid is not limited particularly, and there is no problem at all even if the prosthesis device is any of an inlay, a laminate, a crown a bridge and the like. Therefore, a shape of a dental zirconia for cutting and machining which is cut and machined for preparing a prosthesis device is not limited particularly, and any dental zirconia for cutting and machining can be used even if the dental zirconia for cutting and machining has any shape such as a block shape corresponding to an inlay, a laminate, a crown and the like and a disk shape corresponding to a bridge. In addition, it is more preferable that a dental zirconia for cutting and machining which has a multilayered structure and has a block shape or a disk shape for preparing more aesthetic prosthesis device by applying the opaque imparting liquid.

In addition, the opaque imparting liquid may be prepared by mixing all components. A liquid form which is in a state with the fluidity is preferable. The state of the opaque imparting liquid is not limited particularly and specific example of the states includes a state where all components are uniformly compatible with, a state where the opaque imparting liquid is divided into a plurality of layers and a state where a specific component is separated and precipitated. There is no problem particularly as long as the whole is in a uniform state by an operation such as shaking before use. Among them, a liquid form which has fluidity and low viscosity and in which all components are compatible with is preferable from a point of view that the opaque imparting liquid permeates after applying the opaque imparting liquid to a prosthesis device. For example, the opaque imparting liquid can only adjust transparency to impart opaque property only to a portion where the opaque imparting liquid is applied by applying to the inner surface of the prosthesis device cut and machined from a dental zirconia for cutting and machining.

### [Examples]

The present invention is described in more detail and specifically with reference to Examples. However, the present invention is not limited to Examples.

The opaque imparting liquids containing components shown in following Examples and Comparative Examples were prepared as the zirconia opaque imparting liquid. The preparation was performed by mixing each reagents with a solvent for one hour. Semi-sintered zirconia piece having a dimension of 10mm × 10mm × 1mm was cut out from "SHOFU DISK ZR Lucent FA super light color" manufactured by SHOFU INC. as a zirconia disk imparted with opaque property. Thereafter, the opaque imparting liquids described in Examples and Comparative Examples were applied by a writing brush to the piece. The piece was sintered by the method described in the manual. In addition, opaque property was evaluated by calculating based on the visible light transmissivity in case of not applying the opaque material when the visible light transmissivity in case of not applying the opaque materials was 100. For the application property of the opaque imparting liquid was evaluated by ∘, Δ, ×. The rating criteria were as follow:
∘: Applicable by writing brush.
Δ: Viscosity was slightly low and it was hard to apply.
×: Not cling to writing brush and it was hard to apply.

In the measurement of the bending test body, test specimens having dimensions of a width of 4.8mm, thickness of 1.6 mm and length 20 mm were prepared by cutting and machining "SHOFU DISK ZR Lucent FA super light color" manufactured by SHOFU INC. as the dental zirconia for cutting and machining. The opaque imparting liquids described in Examples and Comparative Examples were applied on only one side of the test specimen. Thereafter, the test specimen was sufficiently dried at 80° C for one hour. Further, test specimen was sintered by retaining at 1450° C of final retaining temperature for two hours according to the method described in the manual. Three point bending test was performed for the test specimen so that tensile stress was applied the surface applied with the opaque imparting liquid. In addition, the test method was based on ISO 6872-2015.

In addition, in comparative example 1, measurement was performed without the opaque imparting liquid.

**[Table 1]**

| | | Example 1 | Example 2 | Example 3 | Example 4 | Example 5 | Example 6 | Example 7 |
|---|---|---|---|---|---|---|---|---|
| Component (a) | Aluminum nitrate | 20.0 | | | 30.0 | | | |
| | Aluminum acetate | | 30.0 | | | 20.0 | | |
| | lanthanum acetate | | | 20.0 | | | 30.0 | |
| | aluminum ethylenediamine tetraacetate | | | | | | | 200 |
| Component (b) | Water | 77.9 | 67.9 | 77.9 | 67.9 | 77.9 | 67.9 | 77.9 |
| Component (c) | Glveerin | 2.0 | | 2.0 | 2.0 | | 2.0 | 2.0 |
| | Polyethylene glycol | | 2.0 | | | 2.0 | | |
| Coloring material | Red cabbage pigment | 0.1 | 0.1 | 0.1 | 0.1 | 0.1 | 0.1 | 0.1 |

| | | Example 8 | Example 9 | Example 10 | Example 11 | Example 12 | Example 13 | Example 14 |
|---|---|---|---|---|---|---|---|---|
| Component (a) | Aluminum nitrate | 30.0 | 200 | 20.0 | | 10.0 | 37.9 | 200 |
| | Aluminum acetate | | | | 30.0 | | | |
| | lanthanum acetate | | | | | | | |
| | aluminum ethylenediamine tetraacetate | | | | | | | |
| Component (b) | Water | 67.9 | 77.9 | 75.0 | 68.9 | 88.9 | 60.0 | 60.0 |
| Component (c) | Glveerin | | | 5.0 | | | | |
| | Polyethylene glycol | 2.0 | 2.0 | | 1.0 | 1.0 | 2.0 | 19.9 |
| Coloring material | Red cabbage pigment | 0.1 | 0.1 | | 0.1 | 0.1 | 0.1 | 0.1 |

| | | Comparative Example 1 | Comparative Example 2 | Comparative Example 3 | Comparative Example 4 |
|---|---|---|---|---|---|
| Component (a) | Aluminum nitrate | 5.0 | 5.0 | 50.0 | 10.0 |
| | Aluminum acetate | | | | |
| | lanthanum acetate | | | | |
| | aluminum ethylenediamine tetraacetate | | | | |
| Component (b) | Water | 929 | 92.9 | 47.9 | 89.9 |
| Component (c) | Glveerin | 2.0 | 2.0 | 2.0 | |
| | Polyethylene glycol | | | | |
| Coloring material | Red cabbage pigment | 0.1 | 0.1 | 0.1 | 0.1 |

| | Example 1 | Example 2 | Example 3 | Example 4 | Example 5 | Example 6 | Example 7 |
|---|---|---|---|---|---|---|---|
| Opaque effect | 55 | 33 | 56 | 36 | 60 | 40 | 60 |
| Application property | A | A | A | A | A | A | A |
| Bending strength [MPa] | 995 | 960 | 998 | 940 | 970 | 966 | 890 |

| | Example 8 | Example 9 | Example 10 | Example 11 | Example 12 | Example 13 | Example 14 |
|---|---|---|---|---|---|---|---|
| Opaque effect | 65 | 45 | 46 | 61 | 45 | 66 | 51 |
| Application property | A | A | A | A | A | A | A |
| Bending strength [MPa] | 910 | 995 | 996 | 905 | 906 | 910 | 920 |

| | Comparative Example 1 | Comparative Example 2 | Comparative Example 3 | Comparative Example 4 |
|---|---|---|---|---|
| Opaque effect | 100 | 95 | 30 | 95 |
| Application property | A | A | A | C |
| Bending strength [MPa] | 1016 | 1019 | 676 | 995 |

### (Consideration of Examples)

An opaque effect was sufficiently exhibited in Examples 1-14 in comparison with Comparative example 1. When the opaque effect was 70 or less, the application effect was exhibited and it was useful clinically. On the other hand, in Comparative example 2, the amount of the component (a) was little and the opaque effect was few. In addition, in Comparative example 3, although the opaque effect was recognized, the bending strength remarkably decreased. In comparative example 4, application property was wrong and it was hard to use clinically.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this disclosure without difficulty.

### [Industrial Applicability]

The present invention relates to an opaque imparting method to impart an opaque property on a zirconia ceramic prosthesis device after sintering by applying to a prosthesis device cut and machined from a dental zirconia for cutting and machining by using a CAD/CAM system in a dental field, and can thus be applied in industries.

## Claims

1. An opaque imparting method comprising;
applying an opaque imparting liquid only to an inner surface of a prosthesis device cut and machined from a dental zirconia for cutting and machining, wherein
the opaque imparting liquid contains
(a) 10 to 39 wt.% of a water-soluble aluminum compound and/or a water-soluble lanthanum compound,
(b) 60 to 89 wt.% of water, and
(c) 1 to 20 wt.% of an organic solvent.

2. The opaque imparting method according to claim 1, wherein
the prosthesis device cut and machined from the dental zirconia for cutting and machining is a crown or a bridge.

3. The opaque imparting method according to claim 1 or 2, wherein
the organic solvent (c) contains any of alcohols, polyol and glycol ethers.

4. The opaque imparting method according to any one of claims 1 to 3, wherein
the water-soluble aluminum compound and/or the water-soluble lanthanum compound (a) contains aluminum acetate and/or lanthanum acetate.

5. The opaque imparting method according to any one of claims 1 to 4, wherein
the dental zirconia for cutting and machining is colored by a zirconia powder where iron, erbium, cobalt or the like is solid-solved.

## Patentansprüche

1. Opazität verleihendes Verfahren, umfassend;
Auftragen einer Opazität verleihenden Flüssigkeit ausschließlich auf eine innere Oberfläche einer aus zahnmedizinischem Zirkoniumoxid zum Schneiden und Bearbeiten geschnittenen und bearbeiteten Prothesenvorrichtung, wobei die Opazität verleihende Flüssigkeit enthält:
(a) 10 bis 39 Gew.-% einer wasserlöslichen Aluminiumverbindung und/oder einer wasserlöslichen Lanthanverbindung,
(b) 60 bis 89 Gew.-% Wasser, und
(c) 1 bis 20 Gew.-% eines organischen Lösungsmittels.

2. Opazität verleihendes Verfahren gemäß Anspruch 1, wobei
die aus dem zahnmedizinischen Zirkoniumoxid zum Schneiden und Bearbeiten geschnittene und bearbeitete Prothesenvorrichtung eine Krone oder eine Brücke ist.

3. Opazität verleihendes Verfahren gemäß Anspruch 1 oder 2, wobei
das organische Lösungsmittel (c) einen der folgenden Stoffe enthält: Alkohole, Polyol und Glykolether.

4. Opazität verleihendes Verfahren gemäß einem der Ansprüche 1 bis 3, wobei
die wasserlösliche Aluminiumverbindung und/oder die wasserlösliche Lanthanverbindung (a) Aluminiumacetat und/oder Lanthanacetat enthält.

5. Opazität verleihendes Verfahren gemäß einem der Ansprüche 1 bis 4, wobei
Das zahnmedizinische Zirkoniumoxid zum Schneiden und Bearbeiten durch ein Zirkoniumoxidpulver gefärbt ist, in dem Eisen, Erbium, Kobalt oder dergleichen fest gelöst ist.

## Revendications

1. Procédé destiné à conférer de l'opacité comprenant:
appliquer un liquide conférant de l'opacité uniquement sur une surface interne d'un dispositif de prothèse découpé dans et usiné à partir d'une zircone dentaire pour la découpe et l'usinage, dans lequel le liquide conférant de l'opacité contient
(a) entre 10 et 39 % en poids d'un composé d'aluminium soluble dans l'eau et/ou d'un composé de lanthane soluble dans l'eau,
(b) entre 60 et 89 % en poids d'eau, et
(c) entre 1 et 20 % en poids d'un solvant organique.

2. Procédé destiné à conférer de l'opacité selon la revendication 1, dans lequel
le dispositif de prothèse découpé dans et usiné à partir de la zircone dentaire pour la découpe et l'usinage est une couronne ou un pont.

3. Procédé destiné à conférer de l'opacité selon la revendication 1 ou 2, dans lequel
le solvant organique (c) contient l'un quelconque parmi les alcools, le polyol et les éthers de glycol.

4. Procédé destiné à conférer de l'opacité selon l'une quelconque des revendications 1 à 3, dans lequel
le composé d'aluminium soluble dans l'eau et/ou le composé de lanthane soluble dans l'eau (a) contient de l'acétate d'aluminium et/ou de l'acétate de lanthane.

5. Procédé destiné à conférer de l'opacité selon l'une quelconque des revendications 1 à 4, dans lequel
la zircone dentaire pour la découpe et l'usinage est colorée par une poudre de zircone dans laquelle du fer, de l'erbium, du cobalt ou similaire est dissous sous forme solide.
